# EUROPEAN PATENT APPLICATION

(11) **EP 3 360 459 A1**
(43) Date of publication of application: **15.08.2018**
(21) Application number: 16853410.5
(22) Date of filing: 16.09.2016
(51) Int. Cl.: A61B 1/00, G02B 23/24

(54) **ENDOSCOPE SYSTEM AND OPERATION METHOD FOR ENDOSCOPE SYSTEM**

(30) Priority: 07.10.2015 JP 2015199393
(71) Applicant: Fujifilm Corporation, Minato-ku Tokyo 106-8620 (JP)
(72) Inventor: KAMON Shumpei, Ashigarakami-gun Kanagawa 258-8538 (JP)
(74) Representative: Klunker IP Patentanwälte PartG mbB
(86) International application number: PCT/JP2016/077437
(87) International publication number: WO 2017/061256

(57) **Abstract**

Provided are an endoscope system and a method of operating the endoscope system that can accurately calculate a diagnostic support parameter more useful than individual blood vessel index values, using the plurality of blood vessel index values, in accordance with observation conditions. An image processing unit includes an image acquisition unit, a blood vessel index value acquisition unit, and a blood vessel parameter calculation unit. The image acquisition unit acquires an endoscopic image captured by an endoscope. The blood vessel index value acquisition unit acquires a first blood vessel index value in a first observation condition and a second blood vessel index value in a second observation condition, as blood vessel index values of blood vessels of an observation target, using the endoscopic image acquired by the image acquisition unit. The blood vessel parameter calculation unit calculates a blood vessel parameter, using the first blood vessel index value and the second blood vessel index value.

## Description

### BACKGROUND OF THE INVENTION

### 1. Field of the Invention

The present invention relates to an endoscope system and a method of operating the endoscope system for calculating data, such as numerical values provided for diagnosis using an endoscopic image captured by an endoscope.

### 2. Description of the Related Art

In the medical field, diagnosis using an endoscope system including a light source device, an endoscope, and a processor device has been performed widely. In the endoscope system, an observation target is irradiated with illumination light emitted from the light source device via the endoscope, and the processor device produces an image of the observation target on the basis of an image signal obtained by imaging the observation target under illumination with the illumination light. By displaying the image on the monitor, a doctor can perform diagnosis while viewing this image on a monitor.

Additionally, in the diagnosis using the endoscope system, in addition to the normal observation of observing the observation target under illumination with white illumination light, for example, the living body information observation of ascertaining the state of the observation target is also performed using information on the fluorescence emitted by the observation target as illustrated in JP2008-229025A, JP2006-061683A, or living body information, including information on blood vessels, such as the thickness or density of blood vessels of the observation target as illustrated in JP2011-217798A.

### SUMMARY OF THE INVENTION

Even in the living body information, a blood vessel index value obtained by quantifying "information on blood vessels", such as the thickness or density of blood vessels, is information that is particularly useful in a case where a doctor performs diagnosis. However, a doctor does not perform diagnosis on the basis of one blood vessel index value, but comprehensively performs diagnosis in consideration of a plurality of blood vessel index values in many cases. For this reason, in recent years, a new attempt to support diagnosis is made by the endoscope system by calculating more direct and useful information or the like than a single blood vessel index value, using a plurality of blood vessel index values, and presenting the information to the doctor.

However, it is often difficult to accurately acquire the blood vessel index values in a case where the ways in which blood vessels appear within an image vary with a change in observation condition, such as an observation distance. For example, since thin blood vessels appear clearly in a case where an observation distance is short, a blood vessel index value can be accurately acquired regarding the thin blood vessels. However, fewer thin blood vessels than in a case where the observation distance is short appear in a case where the observation distance is long. Thus, it is often difficult to accurately acquire the blood vessel index value regarding the thin blood vessels.

Regarding this problem, JP2008-229025A, JP2006-061683A among JP2008-229025A, JP2006-061683A, and JP2011-217798A disclose a method of correcting fluorescence in accordance with the observation distance. However, in this correction method, the brightness of a fluorescence image is adjusted in accordance with the observation distance, and a high-definition image in which blood vessels appear clearly cannot be obtained. In a case where the blood vessels do not appear clearly, it is difficult to accurately acquire the blood vessel index value. For this reason, in this case, it is difficult to accurately calculate more direct and useful information or the like than the single blood vessel index value, using the plurality of blood vessel index values.

An object of the invention is to provide an endoscope system and a method of operating the endoscope system for accurately calculating a diagnostic support parameter (hereinafter referred to as a blood vessel parameter) more useful than individual blood vessel index values, in conformity with the actual conditions of diagnosis utilizing an endoscopic image, using the plurality of blood vessel index values, in accordance with observation conditions.

An endoscope system of the invention comprises an image acquisition unit that acquires an image obtained by imaging an observation target by an endoscope; a blood vessel index value acquisition unit that acquires a first blood vessel index value in a first observation condition and a second blood vessel index value in a second observation condition different from the first observation condition, as blood vessel index values of blood vessels of an observation target from the image, thereby acquiring at least two or more blood vessel index values including the first blood vessel index value and the second blood vessel index value; and a blood vessel parameter calculation unit that calculates a blood vessel parameter, using the first blood vessel index value and the second blood vessel index value.

It is preferable that the blood vessel parameter calculation unit performs calculation while performing weighting based on the first blood vessel index value and the second blood vessel index value on the first blood vessel index value and the second blood vessel index value.

It is preferable that the blood vessel index value acquisition unit acquires at least three or more blood vessel index values including the first blood vessel index value, the second blood vessel index value, and a specific blood vessel index value and the blood vessel parameter calculation unit performs calculation while performing weighting on only blood vessel index values other than the specific blood vessel index value among the three or more blood vessel index values.

It is preferable that the endoscope system further comprises a blood vessel index value correction unit that corrects the first blood vessel index value with a first correction value based on the first observation condition and corrects the second blood vessel index value with a second correction value based on the second observation condition, and the blood vessel parameter calculation unit performs calculation while performing weighting on the first blood vessel index value corrected with the first correction value and the second blood vessel index value corrected with the second correction value.

It is preferable that the endoscope system further comprises a distance acquisition unit that acquires a first observation distance, which is an observation distance from the observation target, and a second observation distance different from the first observation distance, from the image, and the first correction value is a correction value in a case where the first observation condition is the first observation distance, and wherein the second correction value is a correction value in a case where the second observation condition is the second observation distance.

It is preferable that the blood vessel parameter calculation unit calculates the blood vessel parameter by four arithmetic operations.

It is preferable that the endoscope system further comprises a determination unit that determines a state of a mucous membrane of the observation target, using the blood vessel parameter.

It is preferable that the determination unit determines the state of the mucous membrane of the observation target as any of three or more types of states including normality, adenoma, and cancer, using the blood vessel parameter.

It is preferable that the determination unit determines the state of the mucous membrane of the observation target as any one of states including normality, hyperplastic polyp, SSA/P, adenoma, laterally spreading tumor, and cancer, using the blood vessel parameter.

It is preferable that the blood vessel index value acquisition unit acquires at least two or more blood vessel index values from a number of blood vessels, a number of branches of blood vessels, a branch angle of blood vessels, a distance between branch points, a number of intersections of blood vessels, a thickness of blood vessels, a change in thickness of blood vessels, a degree of complexity of a change in thickness of blood vessels, a length of blood vessels, an interval of blood vessels, a depth of blood vessels, a height difference of blood vessels, an inclination of blood vessels, an area of blood vessels, a density of blood vessels, a contrast of blood vessels, a color of blood vessels, a change in color of blood vessels, a degree of meandering of blood vessels, a blood concentration of blood vessels, a degree of oxygen saturation of blood vessels, a proportion of arteries, a proportion of veins, a concentration of input a coloring agent, a traveling pattern of blood vessels, or a blood flow rate of blood vessels.

It is preferable that the first blood vessel index value is the same type as the second blood vessel index value, and the values are different from each other.

It is preferable that the first blood vessel index value is of a type different from the second blood vessel index value.

It is preferable that the first observation condition and the second observation condition are at least any one of an observation distance from the observation target, a zoom magnification factor of the endoscope, an observation mode in which special light is used, or a coloring agent dispersed in the observation target.

A method of operating an endoscope system of the invention comprises a step of acquiring an image obtained by imaging an observation target by an endoscope by an image acquisition unit; a step of acquiring a first blood vessel index value in a first observation condition and a second blood vessel index value in a second observation condition different from the first observation condition, as blood vessel index values of blood vessels of an observation target from the image, thereby acquiring at least two or more blood vessel index values including the first blood vessel index value and the second blood vessel index value by a blood vessel index value acquisition unit; and a step of calculating a blood vessel parameter, using the first blood vessel index value and the second blood vessel index value, by a blood vessel parameter calculation unit.

According to the endoscope system and a method of operating the endoscope system of the invention, it is possible to accurately calculate a blood vessel parameter, using a plurality of blood vessel index values, in accordance with observation conditions.

### BRIEF DESCRIPTION OF THE DRAWINGS

Fig. 1 is an external view of an endoscope system of a first embodiment.
Fig. 2 is a block diagram illustrating functions of the endoscope system.
Fig. 3 is an explanatory view illustrating a special observation image of a non-magnified observation distance.
Fig. 4 is an explanatory view illustrating a special observation image of a magnified observation distance.
Fig. 5 is an explanatory view illustrating calculation of a blood vessel parameter.
Fig. 6 is an explanatory view illustrating a monitor that displays the blood vessel parameter.
Fig. 7 is a flowchart illustrating operation of the endoscope system of the first embodiment.
Fig. 8 is an explanatory view illustrating calculation of the blood vessel parameter using specific blood vessel index values.
Fig. 9 is a block diagram illustrating an image processing unit of a second embodiment.
Fig. 10 is a block diagram illustrating an image processing unit of a third embodiment.
Fig. 11 is an explanatory view illustrating an endoscopic image captured after an endoscope is angled.
Fig. 12 is a block diagram illustrating an image processing unit of a fourth embodiment.
Fig. 13 is an explanatory view illustrating the monitor that displays a determination result obtained by a determination unit.
Fig. 14 is a block diagram illustrating functions of an endoscope system of a fifth embodiment.
Fig. 15 is a plan view of a rotation filter.
Fig. 16 is a schematic view of a capsule endoscope of a sixth embodiment.

### DESCRIPTION OF THE PREFERRED EMBODIMENTS

### [First Embodiment]

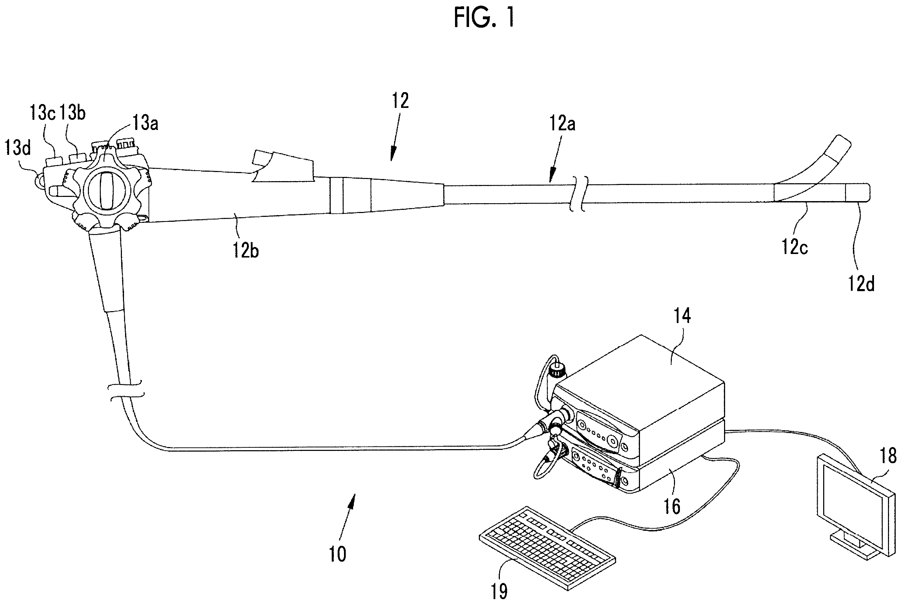

As illustrated in Fig. 1, an endoscope system 10 has an endoscope 12, a light source device 14, a processor device 16, a monitor 18, and a console 19. The endoscope 12 is optically connected to the light source device 14 and is electrically connected to the processor device 16. The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 13a of the operating part 12b, the bending part 12c makes a bending motion. The distal end part 12d is directed in a desired direction by this bending motion.

Additionally, the operating part 12b is provided with a still image acquisition unit 13b used for operating the acquisition of still images, a mode switching unit 13c used for operating the switching of observation modes, and a zooming operating unit 13d used for operating the change of a zoom magnification factor, in addition to the angle knob 13a. In the still image acquisition unit 13b, a freeze operation of displaying a still image of an observation target on the monitor 18, and release operation of saving the still image in a storage are possible.

The endoscope system 10 has a normal mode and a special mode as the observation modes. In a case where an observation mode is the normal mode, the light source device 14 emits white light as illumination light. In a case where an observation mode is the special mode, the light source device 14 emits light (hereinafter referred to as narrowband light) having a specific wavelength range that is a narrower wavelength range than that of the white light as the illumination light.

The processor device 16 is electrically connected to the monitor 18 and the console 19. The monitor 18 outputs and displays an image of the observation target, information accompanying the image, and the like. The console 19 functions as a user interface that receives input operations, such as designation or the like of a region of interest (ROI) and function setting.

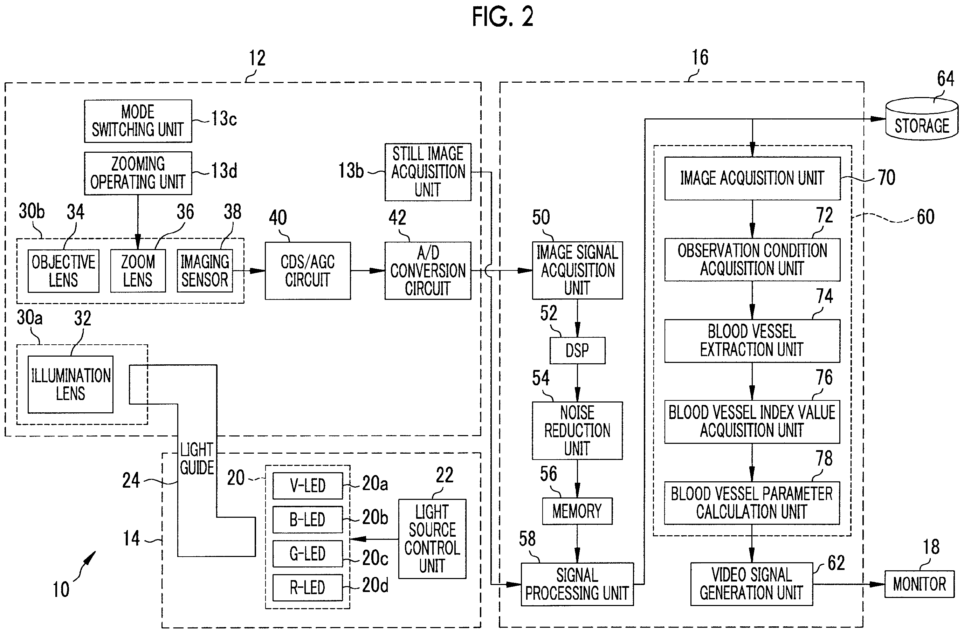

As illustrated in Fig. 2, the light source device 14 includes a light source 20 that emits the illumination light to be used for illumination of the observation target, and a light source control unit 22 that controls the light source 20. The light source 20 is, for example, semiconductor light sources, such as light emitting diodes (LED) in a plurality of colors having different wavelength ranges, a combination of a laser diode and a fluorescent material, a xenon lamp, or the like. Additionally, optical filters that adjust the wavelength ranges of light beams emitted from the LEDs are included in the light source 20. The light source control unit 22 controls the quantity of light emission of the illumination light by ON/OFF of the LEDs and the adjustment of the driving currents or driving voltages of the LEDs. Additionally, the light source control unit 22 controls the wavelength range of the illumination light, for example, by changing the optical filters.

In the present embodiment, the light source 20 has four color LEDs of a violet light emitting diode (V-LED) 20a, a blue light emitting diode (B-LED) 20b, a green light emitting diode (G-LED) 20c, and a red light emitting diode (R-LED) 20d. The V-LED 20a emits purple light V having a wavelength range of 380 nm to 420 nm. The B-LED 20b emits blue light B having a wavelength range of 420 nm to 500 nm. The G-LED 20c emits green light G having a wavelength range of 480 nm to 600 nm. The R-LED 20d emits red light R having a wavelength range of 600 nm to 650 nm. In addition, center wavelengths and peak wavelengths of the respective color lights may be the same as each other or may be different from each other.

The light source control unit 22 independently controls ON/OFF of the respective LEDs, the amount of light emission at the time of ON, and the like, thereby adjusting the light emission timing of illumination light, a light emission period, the quantity of light, and a spectroscopic spectrum. The control of ON and OFF in the light source control unit 22 varies in the respective observation modes. In the case of the normal mode, the light source control unit 22 turns on the V-LED 20a, the B-LED 20b, the G-LED 20c, and the R-LED 20d altogether. For this reason, in the normal mode, white light is emitted by emitting all the purple light V, the blue light B, the green light G, and red light R. Meanwhile, in the case of the special mode, the light source control unit 22, for example, performs switching between the control of turning on only V-LED 20a, and the control of turning on only B-LED 20b. In the special mode in this case, the purple light V and the blue light B are emitted sequentially.

The illumination light emitted from the light source 20 enters a light guide 24 inserted into the insertion part 12a. The light guide 24 is built in the endoscope 12 and a universal cord, and propagates the illumination light up to the distal end part 12d of the endoscope 12. The universal cord is a cord that connects the endoscope 12, and the light source device 14 and the processor device 16 together. In addition, multimode fiber can be used as the light guide 24. As an example, a fine-diameter fiber cable of which the core diameter is 105 µm, the clad diameter is 125 µm, and a diameter including a protective layer used as an outer cover is φ0.3 to 0.5 mm can be used for the light guide 24.

The distal end part 12d of the endoscope 12 is provided with an illumination optical system 30a and an imaging optical system 30b. The illumination optical system 30a has an illumination lens 32. The observation target is illuminated with the illumination light propagated by the light guide 24 via the illumination lens 32. The imaging optical system 30b has an objective lens 34, a zoom lens 36, and an imaging sensor 38. Various kinds of light, such as reflected light from the observation target, scattered light, and fluorescence enters the imaging sensor 38 via the objective lens 34 and the zoom lens 36. Accordingly, the image of the observation target is formed on the imaging sensor 38. The zoom lens 36 moves freely between a telephoto-end and a wide-end by operating the zooming operating part 13d, and magnifies or reduces the observation target of which the image is to be formed on the imaging sensor 38.

The imaging sensor 38 is a color imaging sensor in which any of color filters in primary colors of R (red), G (green), and B (blue) is provided for each pixel, and image signals of respective RGB colors are output by imaging the observation target. The imaging sensor 38 transmits the output image signals to a CDS/AGC circuit 40. As the imaging sensor 38, a charge coupled device (CCD) imaging sensor, a complementary metal-oxide-semiconductor (CMOS) imaging sensor, or the like is available. Additionally, instead of the imaging sensor 38 provided with the color filters in the primary colors, a complementary color imaging sensor including complementary color filters in C (cyan), M (magenta), Y (yellow), and G (green) may be used. In a case where the complementary color imaging sensor is used, image signals of four colors of CMYG are output. For this reason, the same respective RGB image signals as those in the imaging sensor 38 can be obtained by converting the image signals of four colors of CMYG into image signals of three colors of RGB through color conversion between complementary colors and the primary colors. Additionally, instead of the imaging sensor 38, a monochrome sensor that is not provided with the color filters may be used.

The CDS/AGC circuit 40 performs correlated double sampling (CDS) and automatic gain control (AGC) on analog image signals received from the imaging sensor 38. An analog-to-digital (A/D) conversion circuit 42 converts the analog image signals, which have passed through the CDS/AGC circuit 40, into digital image signals. The A/D conversion circuit 42 inputs the digital image signals after the A/D conversion to the processor device 16.

The processor device 16 includes the image signal acquisition unit 50, a digital signal processor (DSP) 52, the noise reduction unit 54, a memory 56, a signal processing unit 58, an image processing unit 60, and a video signal generation unit 62. For example, the processor device 16 has a central processing unit (CPU), and the CPU functions as the image signal acquisition unit 50, the noise reduction unit 54, the signal processing unit 58, the image processing unit 60, and the video signal generation unit 62.

The image signal acquisition unit 50 acquires the digital image signals from the endoscope 12. The DSP 52 performs, for example, various types of signal processing, such as defect correction processing, offset processing, gain correction processing, linear matrix processing, gamma conversion processing, and demosaicing processing, on the image signals acquired by the image signal acquisition unit 50. In the defect correction processing, a signal of a defective pixel of the imaging sensor 38 is corrected. In the offset processing, a dark current component is removed from the image signals subjected to the defect correction processing, and an accurate zero level is set. In the gain correction processing, a signal level is adjusted by multiplying the image signals subjected to the offset processing by a specific gain.

The linear matrix processing enhances color reproducibility on the image signals subjected to the gain correction processing. In the gamma conversion processing, brightness and saturation of image signals subjected the linear matrix processing are adjusted. By performing the demosaicing processing (also referred to as equalization processing or synchronization processing) on the image signals subjected to the gamma conversion processing, a signal of a color that runs short in each pixel is generated by interpolation. By means of this demosaicing processing, all pixels have signals of respective RGB colors. The noise reduction unit 54 performs noise reducing processing using, for example, a moving average method, a median filter method, or the like on the image signals subjected to the demosaicing processing or the like by the DSP 52, and removes noise. The image signals from which noise is reduced are stored in the memory 56.

The signal processing unit 58 acquires the image signals after the noise reduction from the memory 56. Signal processing, such as color conversion processing, color enhancement processing, and structure enhancement processing, is performed on the acquired image signals, if necessary, and an endoscopic image in color on which the observation target appear is generated. The color conversion processing is the processing of performing conversion of colors on the image signals through 3×3 matrix processing, gradation transformation processing, three-dimensional look-up table (LUT) processing, or the like. The color enhancement processing is performed on the image signals subjected to the color conversion processing. The structure enhancement processing is, for example, the processing of enhancing specific tissue and structure included in the observation target, such as blood vessels and pit patterns, and is performed on the image signals after the color enhancement processing.

The contents of the color conversion processing, the color enhancement processing, and the structure enhancement processing performed by the signal processing unit 58 are different depending on the observation modes. In the case of the normal mode, the signal processing unit 58 performs the above respective various types of signal processing, thereby generating an image (hereinafter referred to as a normal observation image), on which the observation target having natural tones appear, as the endoscopic image. In the case of the special mode, the signal processing unit 58 performs the above various types of signal processing of enhancing at least the blood vessels of the observation target, thereby generating an image (hereinafter referred to as a special observation image), in which the blood vessels are enhanced by expressing the blood vessels of the observation target with differences in color, as the endoscopic image. The signal processing unit 58 inputs the generated endoscopic image to the image processing unit 60.

Additionally, the signal processing unit 58 saves the generated endoscopic image in a storage 64 in a case where a release operation is performed by the still image acquisition unit 13b. The storage 64 is an external storage connected to the processor device 16 by a local area network (LAN) or the like. The storage 64 is, for example, a file server of a system, such as a picture archiving and communication system (PACS), which files the endoscopic image, a network attached storage (NAS), or the like. In addition, in a case where a motion picture of the endoscopic image is displayed on the monitor 18 regardless of the operation of the still image acquisition unit 13b, the motion picture of the endoscopic image may be saved in the storage 64.

The image processing unit 60 performs image processing on the endoscopic image generated by the signal processing unit 58. The image processing unit 60 includes an image acquisition unit 70, an observation condition acquisition unit 72, a blood vessel extraction unit 74, a blood vessel index value acquisition unit 76, and a blood vessel parameter calculation unit 78.

The image acquisition unit 70 acquires the endoscopic image, which is obtained by imaging the observation target by the endoscope 12, from the signal processing unit 58. In the present embodiment, the case of the special mode will be described below. Hence, in the following description, the image acquisition unit 70 acquires the special observation image from the signal processing unit 58.

The observation condition acquisition unit 72 acquires a first observation condition and a second observation condition different from the first observation condition. The term "different" includes a case where the types of observation conditions are different between the first observation condition and the second observation condition, or a case where the types of observation conditions are the same between the first observation condition and the second observation condition, but the ways in which specific blood vessels appear on the observation target are different between an endoscopic image obtained on the first observation condition and an endoscopic image obtained on the second observation condition.

The types of observation conditions capable of being acquired as the first observation condition and the second observation condition by the observation condition acquisition unit 72 are, for example, an observation distance from the observation target, the zoom magnification factor of the endoscope 12, an observation mode in which special light is used, a coloring agent dispersed on the observation target, and the like. The observation distance from the observation target is, for example, the non-magnified observation distance in which the observation distance is a long distance, the magnified observation distance in which the observation distance is a short distance, or the like. The zoom magnification factor of the endoscope 12 is, for example, a low magnification factor at which non-magnified observation is performed, a high magnification factor that allows magnified observation, or the like. The observation mode using special light is, for example, a surface layer blood vessel observation mode capable of observing surface layer blood vessels by using purple light and blue light, a middle deep layer blood vessel observation mode capable of observing middle deep layer blood vessels by using green light and red light, or the like. The coloring agent dispersed on the observation target is, for example, a red-based coloring agent, a blue-based coloring agent, or the like. In the present embodiment, the observation condition acquisition unit 72 acquires the first observation condition and the second observation condition from these four types of observation conditions on the basis of the input operation of the console 19. In addition, the types of observation conditions are not limited only to the above four types.

In addition, the case where the types of observation conditions are different between the first observation condition and the second observation condition is, for example, a case where the observation distance from the observation target is acquired as the first observation condition among the above four types of observation conditions, and the observation mode using the special light is acquired as the second observation condition.

Additionally, the case where the ways in which specific blood vessels appear on the observation target are different between the endoscopic image obtained on the first observation condition and the endoscopic image obtained on the second observation condition is, for example, a case where the types of observation conditions correspond to the observation distance among the above four types of observation conditions, but the non-magnified observation distance at which blood vessels appear while being reduced within the endoscopic image is acquired as the first observation condition, and the magnified observation distance at which blood vessels appear while being magnified within the endoscopic image is acquired as the second observation condition.

In the present embodiment, the observation condition acquisition unit 72 acquires the observation distance from the observation target. For example, the observation condition acquisition unit 72 acquires the non-magnified observation distance, which is an observation distance when a change in the zoom magnification factor is started, as the first observation condition on the basis of the operation of the zooming operating unit 13d. Additionally, the observation condition acquisition unit 72 acquires the magnified observation distance, which is an observation distance when the change in the zoom magnification factor is ended, as the second observation condition on the basis of the operation of the zooming operating unit 13d.

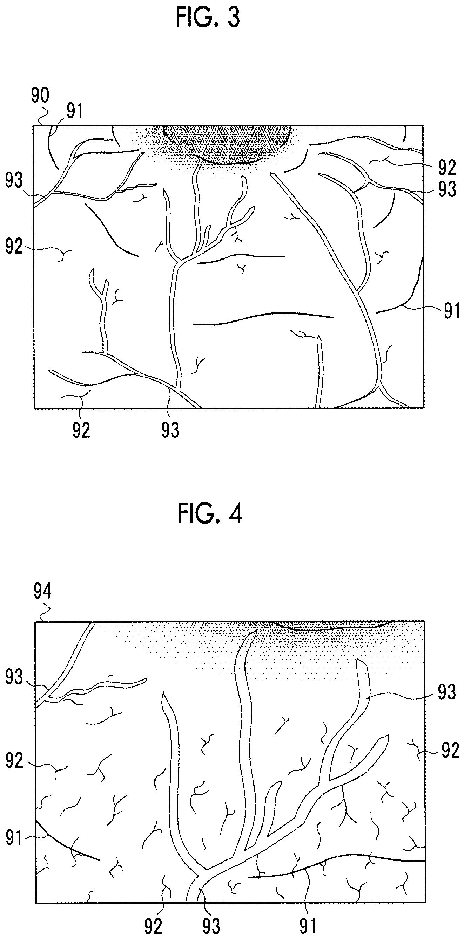

The observation distances can be acquired on the basis of, for example, an exposure amount obtained from an endoscopic image. The observation condition acquisition unit 72 acquires the non-magnified observation distance on the basis of the exposure amount from a special observation image 90 illustrated in Fig. 3 acquired by the image acquisition unit 70, in a case where the change in the zoom magnification factor is started. In the special observation image 90, as well as a shape 91 on a mucous membrane surface of the observation target can be observed, thin surface layer blood vessels 92 that are at a relatively shallow position with the mucous membrane surface as a reference, and thick middle deep layer blood vessels 93 that are at a relatively deep position can be observed. In addition, the surface layer blood vessels 92 are represented by a magenta-based color, and the middle-deep layer blood vessels 93 are represented by a cyan-based color.

Additionally, the observation condition acquisition unit 72 acquires the magnified observation distance on the basis of the exposure amount from a special observation image 94 illustrated in Fig. 4 acquired by the image acquisition unit 70, in a case where the change in the zoom magnification factor is ended. In the special observation image 94, the shape 91 on the mucous membrane surface, the surface layer blood vessels 92 and the middle-deep layer blood vessels 93 can be observed similarly to the special observation image 90 at the non-magnified observation distance.

In a case where the special observation image 90 is compared with the special observation image 94, the observation distance of the special observation image 90 is farther than that of the special observation image 94. Thus, only some surface layer blood vessels 92 among the surface layer blood vessels 92 on the special observation image 94 appear. Hence, the special observation image 90 is a smaller number of surface layer blood vessels 92 than the special observation image 94.

In addition, the observation distances may be acquired by the frequency analysis of an endoscopic image. In a case where the frequency analysis is performed on the special observation image 90, the number of high-frequency components, that is, the number of surface layer blood vessels 92 is small. Thus, the special observation image 90 can be estimated to be an image obtained at the non-magnified observation distance. On the other hand, in a case where the frequency analysis is performed on the special observation image 94, the number of high-frequency components, that is, the number of surface layer blood vessels 92 is large. Thus, the special observation image 94 can be estimated to be an image obtained at the magnified observation distance. Hence, the observation condition acquisition unit 72 can perform the frequency analysis of the special observation image 90, thereby acquiring the non-magnified observation distance, and can perform the frequency analysis of the special observation image 94, thereby acquiring the magnified observation distance.

The blood vessel extraction unit 74 extracts blood vessels of the observation target from the endoscopic image acquired by the image acquisition unit 70. The blood vessel extraction unit 74 extracts blood vessels from the endoscopic image, for example, by a frequency filter or the like. In the present embodiment, since the special observation image 90 at the non-magnified observation distance and the special observation image 94 at the magnified observation distance are acquired, the blood vessel extraction unit 74 extracts blood vessels from the special observation image 90 and the special observation image 94, respectively. In addition, methods of extracting blood vessels are optional. In a case where the observation target is illuminated with illumination light beams having different wavelength ranges by the light source device 14 in addition to the above-described example, blood vessels may be extracted by taking a difference between respective images obtained by capturing the observation target under the illumination with respective illumination light beams. For example, surface layer blood vessels at a shallow position under the mucous membrane or blood vessels at a position shallower than the surface layer blood vessels can be extracted by taking a difference between an image obtained by capturing the observation target under illumination with the purple light V with an image obtained by capturing the observation target under illumination with the blue light B. Additionally, in the present embodiment, the blood vessel extraction unit 74 extracts blood vessels from the overall endoscopic image. However, in a case where the region of interest is specified by the operation of the console 19, blood vessels can be extracted only within the specified region of interest.

The blood vessel index value acquisition unit 76 acquires the blood vessel index values of the blood vessels of the observation target, using the endoscopic image acquired by the image acquisition unit 70. Specifically, the blood vessel index value acquisition unit 76 acquires a first blood vessel index value in the first observation condition, and acquires a second blood vessel index value in the second observation condition.

The blood vessel index values can be sorted into a plurality of types. The types of the blood vessel index values are, for example, the number of blood vessels, the number of branches of blood vessels, the branch angle of blood vessels, a distance between branch points, the number of intersections of blood vessels, the thickness of blood vessels, a change in thickness of blood vessels, the degree of complexity of a change in thickness of blood vessels, the length of blood vessels, the interval of blood vessels, the depth of blood vessels, the height difference of blood vessels, the inclination of blood vessels, the area of blood vessels, the density of blood vessels, the contrast of blood vessels, the color of blood vessels, a change in color of blood vessels, the degree of meandering of blood vessels, the blood concentration of blood vessels, the degree of oxygen saturation of blood vessels, the proportion of arteries, the proportion of veins, the concentration of an input coloring agent, the traveling pattern of blood vessels, the blood flow rate of blood vessels, and the like. In addition, the types of blood vessel index values are not limited only to the above four types.

The number of blood vessels is the number of blood vessels extracted within the overall endoscopic image or the region of interest. The number of blood vessels is calculated, for example, using the number of branch points (the number of branches) of the extracted blood vessels, the number of intersection points (the number of intersections) with other blood vessels, or the like. The branch angle of blood vessels is an angle that two blood vessels form at a branch point. The distance between branch points is a linear distance between a any branch point and its adjacent branch point, or a length along a blood vessel from the any branch point to its adjacent branch point.

The number of intersections of blood vessels is the number of intersection points where blood vessels with different depths under the mucous membrane intersect each other on the endoscopic image. More specifically, the number of intersections of blood vessels is the number of times with which blood vessels at a relatively shallow position under the mucous membrane intersect blood vessels at a relatively deep position.

The thickness (vessel diameter) of the blood vessels is a distance between a blood vessel and a boundary line of the mucous membrane. The thickness (vessel diameter) is obtained, for example, by counting the number of pixels in a lateral direction of an extracted blood vessel through the blood vessel from an edge of the blood vessel. Hence, the thickness of blood vessels is the number of pixels. However, in a case where a capturing distance, a zoom magnification factor, and the like when the endoscopic image is captured are known, the thickness can be converted into the unit of length, such as "µm" if necessary.

The change in thickness of blood vessels is a blood vessel index value regarding variations in thickness of blood vessels, and is also referred to as the degree of aperture inequality. The change in thickness of blood vessels is, for example, the change rate (also referred to as the degree of dilation) of the blood vessel diameter. The change rate of the vessel diameter is obtained by "Change rate (%) of blood vessel diameter = Minimum diameter/Maximum diameter x100", using the thickness (minimum diameter) of the thinnest portion of a blood vessel, and the thickness (maximum diameter) of a thickest portion of a blood vessel.

In addition, in a case where an endoscopic image obtained by capturing the observation target in the past examination and an endoscopic image obtained by capturing the same observation target in subsequent new examination, a time change in thickness of the same blood vessels extracted from the endoscopic image obtained in the subsequent new examination with respect to the thickness of blood vessels extracted from the endoscopic image obtained in the past examination may be used as the change in thickness of blood vessels.

Additionally, the percentage of a smaller-diameter part or the percentage of a larger-diameter part may be calculated as the change in thickness of blood vessels. The smaller-diameter part is a portion having a thickness equal to or smaller than a threshold value. The larger-diameter part is a portion having a thickness greater than the threshold value. The percentage of the smaller-diameter part is obtained by "Percentage (%) of smaller-diameter part = Length of smaller-diameter part/Length of blood vessel × 100 "
Similarly, the percentage of the larger-diameter part is obtained by "Percentage (%) of larger-diameter part = Length of larger-diameter part/Length of blood vessel × 100"

The degree of complexity of the change in thickness of blood vessels (hereinafter referred to as "the degree of complexity of a thickness change") is a blood vessel index value representing how complicated the change is in a case where the thickness of blood vessels changes, and is a blood vessel index value calculated by combining a plurality of blood vessel index values (that is, the change rate of the vessel diameter, the percentage of the smaller-diameter part, or the percentage of the larger-diameter part) representing the change in thickness of blood vessels. The degree of complexity of the thickness change can be obtained, for example, by the product of the change rate of the vessel diameter and the percentage of the smaller-diameter part.

The length of blood vessels is the number of pixels counted in a longitudinal direction of an extracted blood vessel.

The interval of blood vessels is the number of pixels representing the mucous membrane between edges of extracted blood vessels. In a case where there is one extracted blood vessel, the interval of blood vessels does not have a value.

The depth of blood vessels is measured on the basis of the mucous membrane (more specifically, the surface of the mucous membrane). The depth of blood vessels with this mucous membrane as a reference can be calculated, for example, on the basis of the color of blood vessels. In the case of the special observation image, the surface layer blood vessel at a position near the surface of the mucous membrane is represented by the magenta-based color and a blood vessel at a position distant from the surface of the mucous membrane (a deep position under the mucous membrane) is represented by the cyan-based color. Thus, the blood vessel index value acquisition unit 76 calculates the middle-deep layer blood vessels with the mucous membrane as a reference for each pixel, on the basis of the balance between signals in respective colors of R, G, and B of pixels extracted as blood vessels.

The height difference of blood vessels is the magnitude of a difference in depth of a blood vessel. For example, the height difference of one blood vessel to be observed is obtained from a height difference between the depth (the maximum depth) of the deepest location of this blood vessel, and the depth (minimum depth) of the shallowest location. The height difference is zero in a case where the depth is constant.

The inclination of blood vessels is the change rate of the depth of a blood vessel, and is calculated using the length of the blood vessel and the depth of the blood vessel. That is, the inclination of blood vessels is obtained by "Inclination of blood vessel = Length of blood vessel/Length of blood vessel". In addition, a blood vessel may be divided into a plurality of sections, and the inclination of the blood vessel may be calculated in each section.

The area of blood vessels is the number of pixels extracted as a blood vessel, or a value proportional to the number of the pixels extracted as the blood vessel. The area of blood vessels is calculated regarding the inside of the region of interest, the outside of the region of interest, and the overall endoscopic image.

The density of blood vessels is the ratio of the blood vessels to unit area. A region of a specific size (for example, a region of a unit area) including pixels, from which the density of blood vessels is calculated, approximately at the center thereof is cut out, and the ratio of blood vessels to all the pixels within this region is calculated. By performing this on all the pixels of the region of interest or the overall endoscopic image, the density of blood vessels of each pixel can be calculated.

The contrast of blood vessels is the contrast of the observation target relative to the mucous membrane. The contrast of blood vessels is calculated by, for example, "Y_{V}/Y_{M}" or "(Y^{V} - Y_{M})/(Y_{V} + Y_{M})", using the luminance Y_{V} of blood vessels, and the luminance Y_{M} of the mucous membrane.

The color of blood vessels is respective values of RGB of the pixels representing the blood vessels. The change in color of blood vessels is a difference or a ratio between respective maximum and minimum values of respective RGB values of the pixels representing the blood vessels. For example, the ratio of a maximum value and a minimum value of B values of the pixels representing the blood vessels, the ratio of a maximum value and a minimum value of G values, or the ratio of a maximum value and a minimum value of R values represents the change in color of blood vessels. The color of blood vessels and the change in color of blood vessels may be calculated regarding respective values of cyan, magenta, yellow, green, and the like through conversion into complementary colors.

The degree of meandering of blood vessels is a blood vessel index value representing a range where the blood vessels meander and travel. The degree of meandering of blood vessels is, for example, the area (number of pixels) of a minimum oblong shape including blood vessels from which the degree of meandering is calculated. Additionally, the ratio of the length of a blood vessel to a linear distance between a start point and an end point of the blood vessel may be used as the degree of meandering of blood vessels.

The blood concentration of blood vessels is a blood vessel index value proportional to the amount of hemoglobin that the blood vessels include. Since the ratio (G/R) of the G values to the R values in the pixels representing the blood vessels is proportional to the amount of hemoglobin, the blood concentration can be calculated for each pixel by calculating the value of G/R.

The degree of oxygen saturation of blood vessels is the amount of oxyhemoglobin to the total amount of hemoglobin (the total amount of oxyhemoglobin and reduced hemoglobin). The degree of oxygen saturation can be calculated using an endoscopic image obtained when the observation target is illuminated with light (for example, blue light with a wavelength of about 470 ± 10 nm) of a specific large wavelength range where a difference in light absorption coefficient between the oxyhemoglobin and the reduced hemoglobin is great. Since the B values of the pixels representing the blood vessels has a correlation with the degree of oxygen saturation in a case where the blue light with a wavelength of about 470 ± 10 nm is used, the degree of oxygen saturation of the respective pixels representing the blood vessels can be calculated by using a table or the like in which the B values are correlated with the degree of oxygen saturation.

The proportion of arteries is the proportion of the number of pixels of arteries to the number of pixels of all blood vessels. Similarly, the proportion of veins is the proportion of the number of pixels of veins to the number of pixels of all blood vessels. The arteries and the veins can be distinguished from each other depending on the degree of oxygen saturation. For example, in a case where blood vessels with the degree of oxygen saturation of 70% or more are the arteries, and blood vessels with the degree of oxygen saturation of less than 70% are the veins, extracted blood vessels are divided into the arteries and the veins. Thus, the proportion of the above arteries and the proportion of the veins can be calculated.

The concentration of an input coloring agent is the concentration of a coloring agent dispersed in the observation target or a coloring agent injected into the blood vessels by intravenous injection. The concentration of an input coloring agent is calculated, for example, in the ratio of the pixel values of the color of the coloring agent to the pixel values of pixels other than the color of the coloring agent. For example, in a case where a coloring agent coloring blue color is input, B/G, B/R, or the like represents the concentration of the coloring agent fixed (or temporarily adhered) to the observation target.

The traveling pattern of blood vessels is a blood vessel index value regarding the traveling direction of blood vessels. The traveling pattern of blood vessels is, for example, the average angle (traveling direction) of blood vessels to an arbitrarily set reference line, the dispersion (variations in traveling direction) of angles that blood vessels make with respect to the arbitrarily set reference line, or the like.

The blood flow rate (also referred to blood flow velocity) of blood vessels is the number of red cells that pass through blood vessels per unit time. For example, in a case where an ultrasonic probe is used together via a forceps channel or the like of the endoscope 12, the blood flow rate of blood vessels can be obtained by calculating the Doppler shift frequency of the respective pixels representing the blood vessels of the endoscopic image, using signals obtained by the ultrasonic probe.

The blood vessel index value acquisition unit 76 calculates a blood vessel index values for each pixel of the endoscopic image, thereby acquiring the blood vessel index value. For example, the blood vessel index value of one pixel is calculated using the data of pixels in a predetermined range including a pixel of which the blood vessel index value is to be calculated (for example, a range of 99 × 99 pixels centered on a pixel of which the blood vessel index value is to be calculated). For example, in a case where the thickness of blood vessels is calculated as a blood vessel index value, "the thickness of blood vessels" for each pixel is the statistic amount of the thickness of blood vessels appearing in the above predetermined range. The statistic amount is a so-called basic statistic amount, for example, a maximum value, a minimum value, an average value, a median value, a most frequent value. Additionally, statistic amount other than the exemplified values may be used. For example, a value (the ratio of the maximum value to the minimum value, and the like) calculated using a so-called representative value, such as a maximum value, a minimum value, an average value, a median value, or a most frequent value, or so-called degree of scatter, such as variance, standard deviation, or coefficient of variation, can be used.

In a case where the region of interest is set in a portion of the endoscopic image by the operation of the console 19, the blood vessel index value acquisition unit 76 calculates blood vessel index values within the set region of interest. In a case where the region of interest is not set or in a case where the overall endoscopic image is set as the region of interest, the blood vessel index value acquisition unit 76 calculates blood vessel index values for the overall endoscopic image.

In a case where the region of interest is set, the blood vessel index value acquisition unit 76 calculates the statistic amount of a blood vessel index value of each pixel included in the region of interest, and acquires the value as the blood vessel index value of the region of interest. For example, in a case where the thickness of blood vessels is calculated as a blood vessel index value, "the thickness of blood vessels" of each pixel is calculated as described above, and in a case where the region of interest is set, the statistic amount of "the thickness of blood vessels" of each pixel included in the region of interest is calculated, and one "the thickness of blood vessels" is calculated for one set region of interest. The same applies to a case where the overall endoscopic image is set as the region of interest.

In addition, a statistic amount in a case where a blood vessel index value for each pixel is calculated and a statistic amount in a case where a blood vessel index value of the region of interest is calculated may be the same statistic amount, or may be different from each other. For example, in a case where the thickness of blood vessels for each pixel is calculated, an average value of the thickness of blood vessels appearing in "the predetermined range" is calculated, and then even in a case where the thickness of blood vessels of the region of interest is calculated, an average value of the thickness of blood vessels of each pixel may be calculated, or the most frequent value of the thickness of blood vessels of each pixel may be calculated.

Additionally, the blood vessel index value for each pixel can be omitted depending on the types of blood vessel index values to be calculated, a relationship between a method of calculating the statistic amount in the case where the blood vessel index value for each pixel is calculated and a method of calculating the statistic amount in the case where the blood vessel index value for the region of interest is calculated, or the like. For example, in the case of "the thickness of blood vessels", an average value of the thickness of blood vessels appearing in the region of interest can be set to the thickness of blood vessels of the region of interest.

In the present embodiment, the blood vessel index value acquisition unit 76 acquires the blood vessel index values regarding the surface layer blood vessels 92 extracted from the special observation image 90 and the special observation image 94, respectively, by the blood vessel extraction unit 74. Additionally, the first blood vessel index value is the same type as the second blood vessel index value, and these values are different from each other. For example, both the types of the first blood vessel index value and the second blood vessel index value are the numbers of blood vessels, that is, the numbers of the surface layer blood vessels 92. The number (first blood vessel index value) of the surface layer blood vessels 92 at the non-magnified observation distance is smaller than the number (second blood vessel index value) of the surface layer blood vessels 92 at the magnified observation distance.

In the special observation image 90 obtained at the non-magnified observation distance, only some surface layer blood vessels 92 among the surface layer blood vessels 92 on the special observation image 94 obtained at the magnified observation distance appear. Hence, the number (second blood vessel index value) of the surface layer blood vessels 92 at the magnified observation distance is more accurate than the number (first blood vessel index value) of the surface layer blood vessels 92 at the non-magnified observation distance.

The blood vessel parameter calculation unit 78 calculates a blood vessel parameter, using the first blood vessel index value and the second blood vessel index value. Specifically, the blood vessel parameter calculation unit 78 performs calculation while performing weighting based on the first blood vessel index value and the second blood vessel index value on the first blood vessel index value and the second blood vessel index value, thereby calculating the blood vessel parameter.

The blood vessel parameter is a numerical value calculated using a plurality of blood vessel index values in imitation of the viewpoint of a doctor who comprehensively performs diagnosis in consideration of the plurality of blood vessel index values. Since the blood vessel parameter is calculated by calculating blood vessel index values with mutually different dimensions (units) through calculation, such as addition, there is no physical meaning in the blood vessel parameter, but the blood vessel parameter functions as an index of diagnosis. That is, the blood vessel parameter is different from the blood vessel index values in that the parameter is a value with physical meaning.

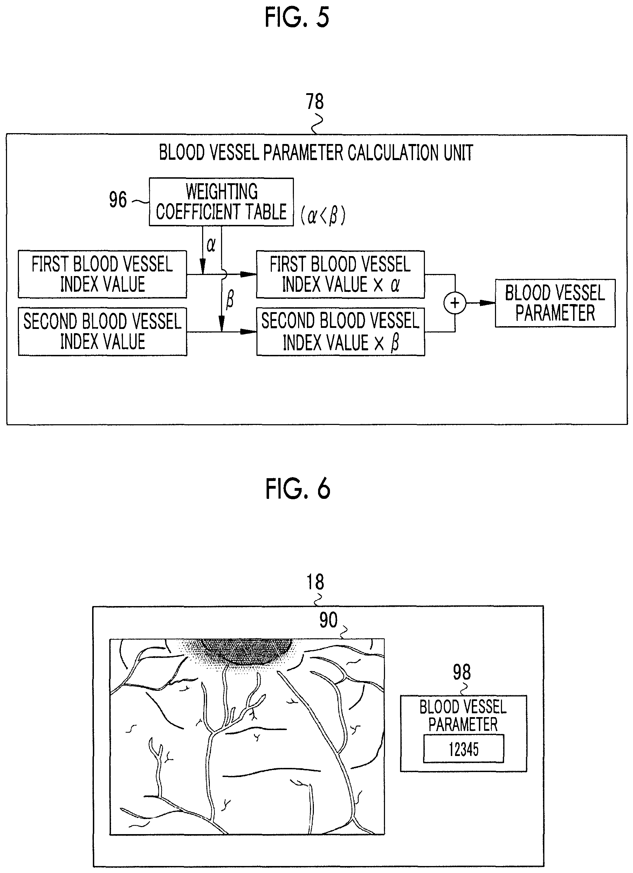

As illustrated in Fig. 5, in the present embodiment, the blood vessel parameter calculation unit 78 performs addition after the first blood vessel index value and the second blood vessel index value are multiplied by weighting coefficients. A weighting coefficient for the number (first blood vessel index value) of the surface layer blood vessels 92 at the non-magnified observation distance is defined as α. The weighting coefficient α is the ratio of the number of surface layer blood vessels 92 at the non-magnified observation distance to the number of surface layer blood vessels 92 at the magnified observation distance. Additionally, a weighting coefficient for the number (second blood vessel index value) of the surface layer blood vessels 92 at the magnified observation distance is defined as β. The weighting coefficient β is the ratio of the number of surface layer blood vessels 92 at the magnified observation distance to the number of surface layer blood vessels 92 at the non-magnified observation distance. Since the number of surface layer blood vessels 92 at the magnified observation distance is more accurate than the number of surface layer blood vessels 92 at the non-magnified observation distance as described above, the blood vessel parameter calculation unit 78 makes the weighting coefficient β for the number of surface layer blood vessels 92 at the magnified observation distance greater than the weighting coefficient α for the number of surface layer blood vessels 92 at the non-magnified observation distance (α < β). The weighting coefficients α and β are stored in a weighting coefficient table 96, and are determined in advance, for example, by machine learning. In addition, the blood vessel parameter calculation unit 78 may calculate the weighting coefficients α and β as described above.

The video signal generation unit 62 converts the endoscopic image processed by the image processing unit 60 into video signals capable of being output and displayed by the monitor 18. Additionally, as illustrated in Fig. 6, the video signal generation unit 62, for example, displays the special observation image 90 on the monitor 18, and displays the blood vessel parameter calculated by the blood vessel parameter calculation unit 78 on a blood vessel parameter display unit 98 set on the monitor 18.

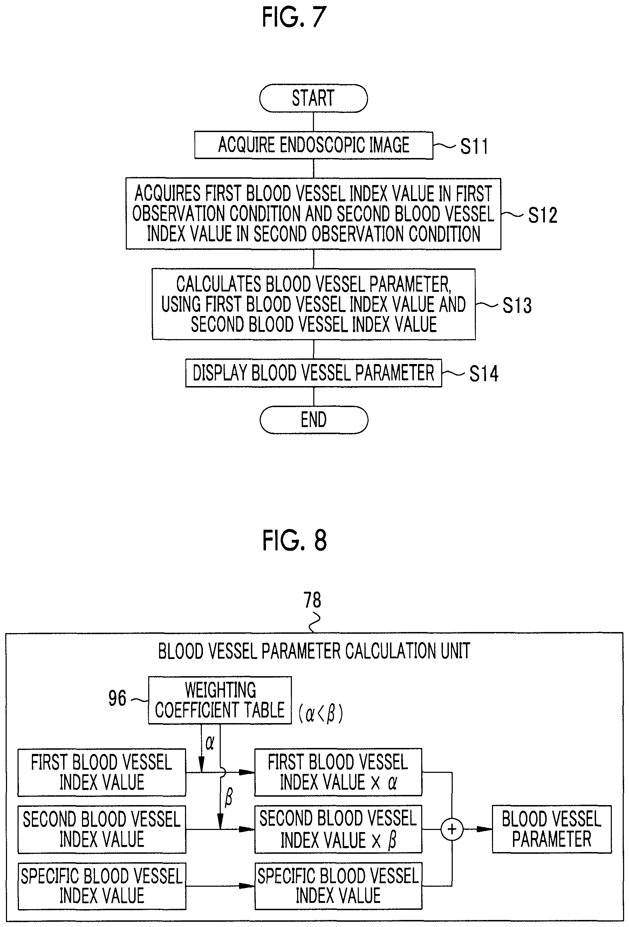

Next, the endoscope system 10 of the invention will be described along a flowchart of Fig. 7.

The image acquisition unit 70 acquires the endoscopic image obtained by imaging the observation target by the endoscope 12 (S11). In the present embodiment, the image acquisition unit 70 acquires the special observation image 90 at the non-magnified observation distance, with the non-magnified observation distance, which is the observation distance when the change in the zoom magnification factor is started, as the first observation condition, in a case where the zooming operating unit 13d is operated. Additionally, the image acquisition unit 70 acquires the special observation image 94 at the magnified observation distance, with the magnified observation distance, which is the observation distance when the change in the zoom magnification factor is ended, as the second observation condition. The blood vessel extraction unit 74 extracts blood vessels of the observation target from the special observation image 90 and the special observation image 94, respectively. Specifically, the blood vessel extraction unit 74 extracts the surface layer blood vessels 92 and the middle-deep layer blood vessels 93, respectively.

The blood vessel index value acquisition unit 76 acquires the first blood vessel index value in the first observation condition and the second blood vessel index value in the second observation condition, using the endoscopic image acquired by the image acquisition unit 70 (S12). Specifically, the blood vessel index value acquisition unit 76 calculates the number of surface layer blood vessels 92 extracted by the blood vessel extraction unit 74, from the special observation image 90 obtained at the non-magnified observation distance. The blood vessel index value acquisition unit 76 acquires the number of surface layer blood vessels 92 at the calculated non-magnified observation distance as the first blood vessel index value. Additionally the blood vessel index value acquisition unit 76 calculates the number of surface layer blood vessels 92 extracted by the blood vessel extraction unit 74, from the special observation image 94 obtained at the magnified observation distance. The blood vessel index value acquisition unit 76 acquires the number of surface layer blood vessels 92 at the calculated magnified observation distance as the second blood vessel index value.

Next, the blood vessel parameter calculation unit 78 calculates the blood vessel parameter, using the first blood vessel index value and the second blood vessel index value (S13). Specifically, the blood vessel parameter calculation unit 78 performs addition after the number of surface layer blood vessels 92 at the non-magnified observation distance, and the number of surface layer blood vessels 92 at the magnified observation distance is multiplied by the ratio of the number of surface layer blood vessels 92 at the non-magnified observation distance and the number of surface layer blood vessels 92 at the magnified observation distance. Then, the blood vessel parameter calculated by the blood vessel parameter calculation unit 78 is displayed on the monitor 18 together with the endoscopic image by the video signal generation unit 62 (S14).

As described above, since the first blood vessel index value in the first observation condition is acquired, the second blood vessel index value in the second observation condition that is different from the first observation condition is acquired, and the blood vessel parameter is calculated using the first blood vessel index value and the second blood vessel index value, a suitable blood vessel parameter according to the observation conditions can be presented to a doctor.

In addition, in the above first embodiment, the blood vessel index value acquisition unit 76 acquires the two blood vessel index values of the first blood vessel index value and the second blood vessel index value. However, two or more blood vessel index values including the first blood vessel index value and the second blood vessel index value may be acquired.

Additionally, although the blood vessel parameter calculation unit 78 calculates the blood vessel parameter using the two blood vessel index values of the first blood vessel index value and the second blood vessel index value, the blood vessel parameter may be calculated using two or more blood vessel index values including the first blood vessel index value and the second blood vessel index value.

Additionally, in the above first embodiment, the blood vessel parameter calculation unit 78 multiplies the first blood vessel index value and the second blood vessel index value by the weighting coefficients, respectively. However, in a case where the blood vessel parameter is calculated using a specific blood vessel index value in addition to the first blood vessel index value and the second blood vessel index value, the blood vessel index values other than the specific blood vessel index value may be multiplied by the weighting coefficients without multiplying the specific blood vessel index value by a weighting coefficient. The "specific blood vessel index value" includes a blood vessel index value regarding certain blood vessels under an observation condition in which it is difficult to accurately observe the blood vessels. For example, the specific blood vessel index value is a blood vessel index value regarding thick blood vessels in a case where the observation distance is short. In an endoscopic image obtained in a case where the observation distance is short, it is difficult to accurately observe a full view due to breaking of some of the thick blood vessels, and the like, as compared to a case where the observation distance is long. In addition, the specific blood vessel index value can also be set by an input operation of the console 19.

As illustrated in Fig. 8, in a case where the specific blood vessel index value is acquired in addition to the first blood vessel index value and the second blood vessel index value, the blood vessel parameter calculation unit 78 multiplies the first blood vessel index value by the weighting coefficient α, multiplies the second blood vessel index value by the weighting coefficient β, does not the specific blood vessel index value by a weighting coefficient, and adds up the respective blood vessel index values. Accordingly, the blood vessel parameter can be calculated more accurately. In addition, similar to the above, the weighting coefficients α and β may be weighting coefficients based on the first blood vessel index value and the second blood vessel index value.

In addition, in a case where the specific blood vessel index value is acquired, the blood vessel parameter calculation unit 78 may not use the specific blood vessel index value for the calculation of the blood vessel parameter.

### [Second Embodiment]

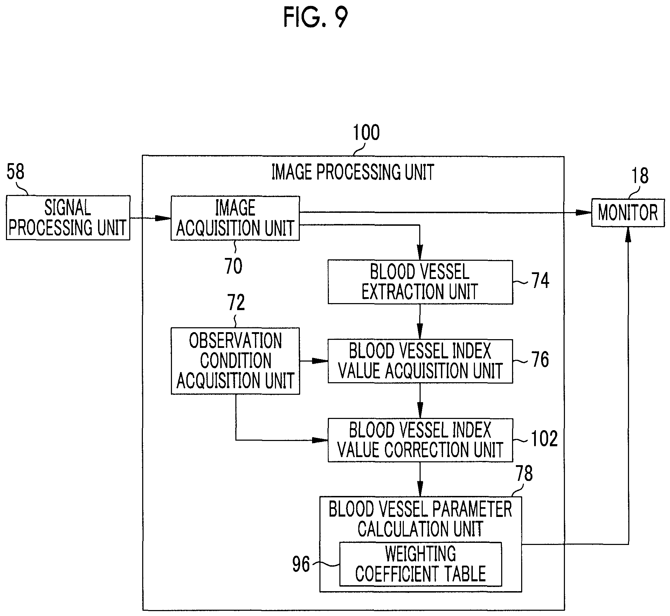

In the above first embodiment, the blood vessel parameter calculation unit 78 multiplies the first blood vessel index value and the second blood vessel index value by the weighting coefficients based on the first blood vessel index value and the second blood vessel index value. However, in a second embodiment, the multiplication of the above weighting coefficients is performed after the first blood vessel index value and the second blood vessel index value are corrected using correction values based on observation conditions, respectively. In this case, as illustrated in Fig. 9, the image processing unit 100 is provided with a blood vessel index value correction unit 102 in addition to the respective components of the image processing unit 60 of the above first embodiment. In the following, description of the same components as those of the above first embodiment will be omitted. In the second embodiment, the CPU of the processor device 16 functions as the image signal acquisition unit 50, the noise reduction unit 54, the signal processing unit 58, the image processing unit 102, and the video signal generation unit 62.

The blood vessel index value correction unit 102 corrects the first blood vessel index value with a first correction value based on the first observation condition and corrects the second blood vessel index value with a second correction value based on the second observation condition. For example, the blood vessel index value correction unit 102 multiplies the density of blood vessels at the non-magnified observation distance by the first correction value. Since the area of a mucous membrane on an image at the magnified observation distance is small as compared with the case of the non-magnified observation distance, the ratio of blood vessels to a unit area is great. For this reason, the density of blood vessels at the non-magnified observation distance is more accurate than the density of blood vessels at the magnified observation distance. Hence, the blood vessel index value correction unit 102 multiplies the density of blood vessels by the first correction value, thereby correcting the density of blood vessels, in the case of the non-magnified observation distance. The first correction value is a greater value than 1.

Additionally, the length of the thick blood vessels at the non-magnified observation distance is multiplied by the second correction value. At the magnified observation distance, it is difficult to accurately observe the full view of the thick blood vessels as described above as compared to the case of the non-magnified observation distance. Thus, it is difficult to acquire the length of the thick blood vessels accurately. For this reason, the length of the thick blood vessels at the non-magnified observation distance is more accurate than the length of the thick blood vessels at the magnified observation distance. Hence, the blood vessel index value correction unit 102 multiplies the length of the thick blood vessels by the second correction value, thereby correcting the length of the thick blood vessels, in the case of the non-magnified observation distance. The second correction value is a greater value than 1.

The blood vessel parameter calculation unit 78 multiplies the density of blood vessels at the non-magnified observation distance corrected with the first correction value by the weighting coefficient based on the density of blood vessels at the non-magnified observation distance and the length of the thick blood vessels at the non-magnified observation distance. Similarly, the blood vessel parameter calculation unit 78 multiplies the length of the thick blood vessels at the non-magnified observation distance corrected with the second correction value by the weighting coefficient based on the density of blood vessels at the non-magnified observation distance and the length of the thick blood vessels at the non-magnified observation distance. Then, the blood vessel parameter calculation unit 78 can add the density of blood vessels corrected with the first correction value and the length of the thick blood vessels corrected with the second correction value together, thereby more accurately calculating the blood vessel parameter.

In addition, the first correction value and the second correction value can be arbitrarily set on the basis of an input operation of the console 19.

### [Third Embodiment]

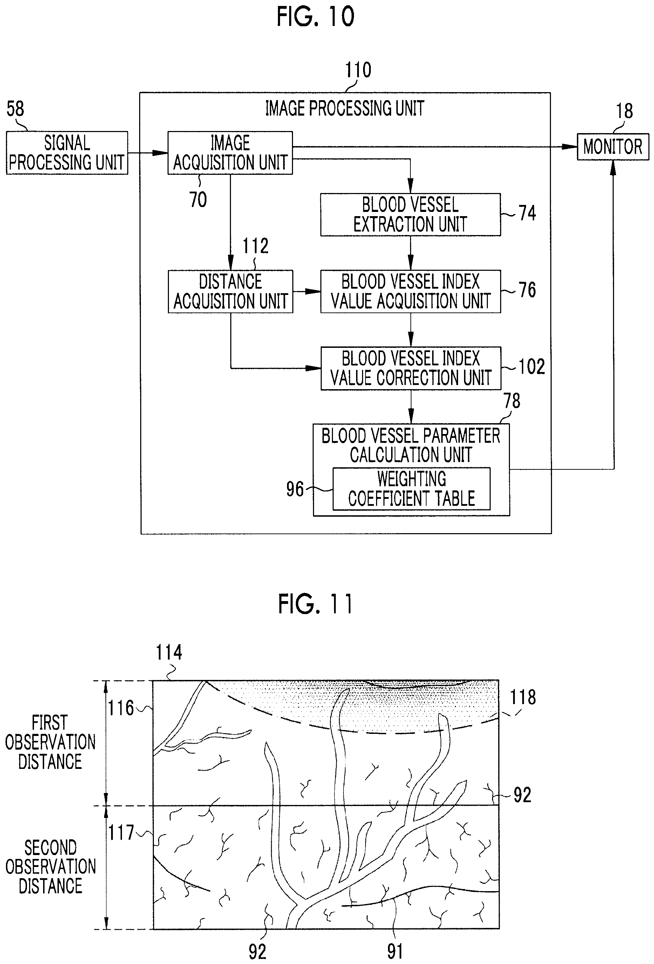

In the above second embodiment, the first observation condition and the second observation condition are acquired by the observation condition acquisition unit 72. However, a first observation distance and a second observation distance are acquired in the third embodiment. In this case, as illustrated in Fig. 10, an image processing unit 110 is provided with a distance acquisition unit 112 instead of the observation condition acquisition unit 72 of the image processing unit 100 of the above second embodiment. In the third embodiment, the CPU of the processor device 16 functions as the image signal acquisition unit 50, the noise reduction unit 54, the signal processing unit 58, the image processing unit 110, and the video signal generation unit 62.

The distance acquisition unit 112 acquires the first observation distance, which is the observation distance from the observation target, and the second observation distance different from the first observation distance, from the endoscopic image acquired by the image acquisition unit 70.

Specifically, the distance acquisition unit 112 splits an endoscopic image 114 illustrated in Fig. 11 into two areas of a first area 116 and a second area 117. The endoscopic image 114 is an image captured in a state where the endoscope 12 is tilted with respect to an axis orthogonal to the surface of the observation target, and the depth of the observation target is shown. A dark part 118 is present in a depth direction in the first area 116. In the dark part 118, the exposure amount becomes equal to or smaller than a threshold value. The second area 117 has a substantially constant exposure amount.

The distance acquisition unit 112 obtains an average exposure amount of the first area 116, thereby obtaining an average observation distance of the first area 116. The distance acquisition unit 112 acquires the obtained average observation distance of the first area 116 as the first observation distance. Similarly, the distance acquisition unit 112 obtains an average exposure amount of the second area 117, thereby obtaining an average observation distance of the second area 117. The distance acquisition unit 112 acquires the obtained average observation distance of the second area 117 as the second observation distance. The average exposure amount in the first area 116 including the dark part 118 is smaller than that in the second area 117. For this reason, the first observation distance is a longer distance than the second observation distance.

In addition, the distance acquisition unit 112 can obtain exposure amounts regarding respective positions within the endoscopic image 114, thereby obtaining the distribution of the exposure amounts within the endoscopic image 114. Also, the distance acquisition unit 112 can estimate the angle of the endoscope 12 on the basis of the distribution of the exposure amounts. The distance acquisition unit 112 may acquire the first observation distance and the second observation distance, using the endoscopic image 114, in a case where the endoscope 12 is estimated to be angled, and may not acquire the first observation distance and the second observation distance, in a case where the endoscope 12 is estimated not be angled.

In the blood vessel index value acquisition unit 76, the first observation distance is set to the first observation condition, and the first blood vessel index value of the first observation distance is acquired. Similarly, the blood vessel index value acquisition unit 76 sets the second observation distance as the second observation condition, and acquires the second blood vessel index value of the second observation distance.

The blood vessel index value acquisition unit 76 acquires, for example, the number of surface layer blood vessels 92 in the first area 116 of the first observation distance. Additionally, the blood vessel index value acquisition unit 76 acquires the number of surface layer blood vessels 92 in the second area 117 of the second observation distance. In addition, in the first area 116 of the first observation distance, only some surface layer blood vessels 92 among the surface layer blood vessels 92 of the second area 117 of the second observation distance appear. Hence, the number of surface layer blood vessels 92 in the second observation distance is more accurate than the number of surface layer blood vessels 92 in the first observation distance.

In the blood vessel index value correction unit 102, the number of surface layer blood vessels 92 in the first observation distance is corrected with the first correction value, and the number of surface layer blood vessels 92 at the second observation distance is corrected with the second correction value. That is, in the third embodiment, the first correction value is a correction value in a case where the first observation condition is the first observation distance. Additionally, the second correction value is a correction value in a case where the second observation condition is the second observation distance. For this reason, even in a case where an endoscopic image captured in a state where the endoscope 12 is angled is used, the blood vessel parameter can be accurately calculated.

### [Fourth Embodiment]

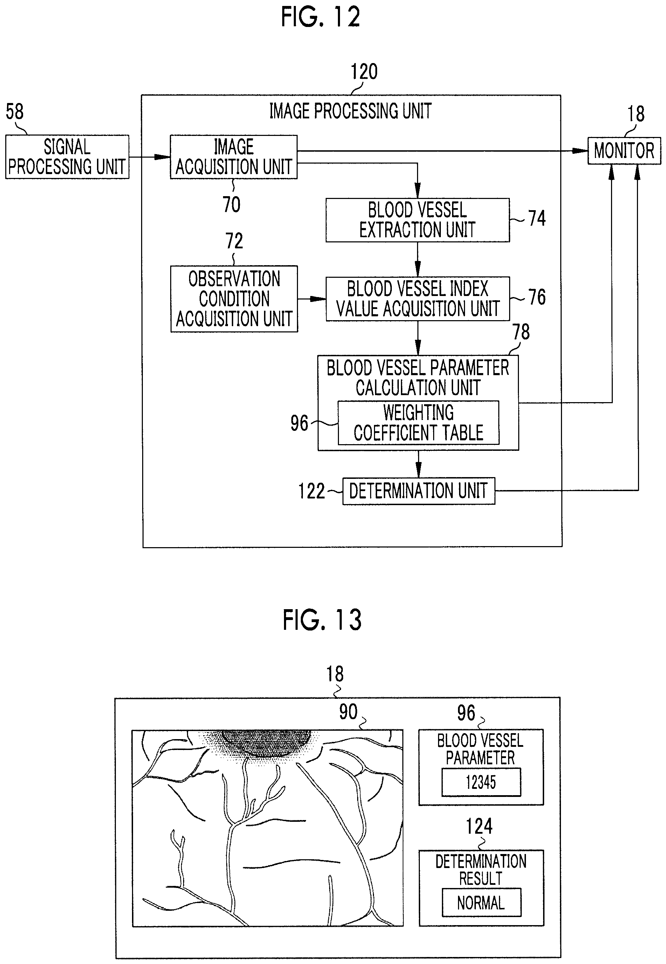

In the above respective embodiments, the blood vessel parameter is displayed on the monitor 18. However, in a fourth embodiment, the state of the mucous membrane of the observation target is determined using the blood vessel parameter, and this determination result is displayed on the monitor 18 together with the blood vessel parameter. As illustrated in Fig. 12, an image processing unit 120 of the fourth embodiment is provided with a determination unit 122 in addition to the respective parts of the image processing unit 60 of the above first embodiment. In the fourth embodiment, the CPU of the processor device 16 functions as the image signal acquisition unit 50, the noise reduction unit 54, the signal processing unit 58, the image processing unit 120, and the video signal generation unit 62.

The determination unit 122 determines the state of the mucous membrane of the observation target, using the blood vessel parameter calculated by the blood vessel parameter calculation unit 78. The "state of the mucous membrane" of the observation target is an overall status as the entire mucous membrane including the blood vessels, for example, is "normal", "adenoma (there is doubt of adenoma)", or "cancer (there is doubt of cancer)". In this case, the determination unit 122 determines the state of the mucous membrane as three types of states of normality, adenoma, and cancer.

For example, it is assumed that a weighting coefficient to be used for the calculation of the blood vessel parameter is set to the balance capable of determining the state of the mucous membrane as any of the three types of states of normality, adenoma, and cancer. In this case, the determination unit 122 compares a numerical value of the blood vessel parameter with a threshold value, thereby determining the state of the mucous membrane. Specifically, in a case where the blood vessel parameter is equal to or smaller than a first threshold value, the determination unit 122 determines the state of the mucous membrane of the observation target as "normal". In a case where the blood vessel parameter is greater than the first threshold value and is equal to or smaller than a second threshold value, the determination unit 122 determines the state of the mucous membrane of the observation target as "adenoma". In a case where the blood vessel parameter is equal than the second threshold value, the determination unit 122 determines the state of the mucous membrane of the observation target as "cancer". As illustrated in Fig. 13, a determination result is displayed on a determination result display unit 124 set on the monitor 18. In this way, diagnosis can be more directly supported by determining the state of the mucous membrane of the observation target, using the blood vessel parameter, and displaying this determination result.

In addition, it is desirable that the determination unit 122 determines the state of the mucous membrane as the three or more types of states including normality, adenoma, and cancer. Particularly, in a case where the state of a mucous membrane of the large intestine is determined, it is desirable to determine the state of the mucous membrane as any of states including normality, hyperplastic polyp (HP), sessile serrated adenoma polyp (SSA/P), traditional serrated adenoma (TSA), laterally spreading tumor (LST), and cancer.

In a case where the determination results of the determination unit 122 are subdivided in this way, it is preferable that the determination unit 122 uses the blood vessel index values in addition to the blood vessel parameter.

In the related art, it was considered that the hyperplastic polyp had a low risk of cancelation and have no necessity for treatment. However, in recent years, since an example in which the SSA/P similar to the hyperplastic polyp was cancerated is also discovered, it is becoming important to distinguish particularly between the hyperplastic polyp and the SSA/P. Meanwhile, in a case where a middle-deep layer blood vessel is crossing the bottom of a thickened mucous membrane believed to be the hyperplastic polyp or SSA/P, it is known that the SSA/P is highly likely to be formed. In a case where the blood vessel parameter is used, the hyperplastic polyp and the SSA/P can be differentiated by the determination unit 122. Moreover, in a case where a determination is performed by combining the blood vessel parameter and the blood vessel index values (the thickness and length of blood vessels), the SSA/P can be differentiated from the hyperplastic polyp with higher probability.

Additionally, in a case where the state of the mucous membrane of the observation target is determined as cancer, it is preferable that the determination unit 122 determines the stage of cancer using the blood vessel parameter. It is preferable to display the stage of cancer determined by the determination unit 122 on the monitor 18. In this way, in a case where the stage is further determined and the result is displayed on the monitor 18 in a case where the state of the mucous membrane of the observation target is determined as cancer, diagnosis can be supported more finely.

Additionally, the blood vessel index values and the weighting coefficients to be used in a case where the blood vessel parameter is calculated may be displayed on the monitor 18. In this case, it is preferable that the blood vessel index values calculated by the blood vessel index value acquisition unit 76 with the weighting coefficients are displayed to match each other. In addition, only the blood vessel index values or the weighting coefficients may be displayed. In this way, it becomes easy for doctors to ascertain the meaning of the blood vessel parameter and the ground for the determination by displaying the blood vessel index values and the weighting coefficients to be used for the calculation of the blood vessel parameter.

In addition, in the above respective embodiments, the first blood vessel index value may be the same type as the second blood vessel index value or may be mutually different values. However, the first blood vessel index value may be of a type different from the second blood vessel index value. For example, the first blood vessel index value may be the density of blood vessels, and the second blood vessel index value may be the depth of blood vessels. By using the plurality of blood vessel index values having different types in this way, diagnosis of a lesion different from a case where the same types of blood vessel index values are used can be supported.

In addition, in the above respective embodiments, the blood vessel parameter is calculated by performing addition after the plurality of blood vessel index values are weighted. However, methods of calculating the blood vessel parameter are optional. For example, the blood vessel parameter may be calculated by the four arithmetic operations. Additionally, the blood vessel parameter may be calculated using other functions. In addition, in this case, the determination unit 122 may compare a numerical value of the blood vessel parameter obtained by the four arithmetic operations with a threshold value, thereby determining the state of the mucous membrane.

In addition, in the above respective embodiments, as the observation modes, the two types of observation modes of the normal mode and the special mode are provided. However, three or more types of observation modes may be provided. For example, the endoscope system 10 has a surface layer blood vessel observation mode and a middle-deep layer blood vessel observation mode in addition to the above two types of observation modes. The observation condition acquisition unit 72 may acquire the surface layer blood vessel observation mode as the first observation condition, and may acquire the middle-deep layer blood vessel observation mode as the second observation condition.

In the Surface layer blood vessel observation mode, purple light and blue light are emitted as the illumination light. In an endoscopic image obtained in the surface layer blood vessel observation mode, the surface layer blood vessels can be observed clearly. In the middle-deep layer blood vessel observation mode, green light and red light are emitted as the illumination light. In the endoscopic image obtained in the middle-deep layer blood vessel observation mode, the middle-deep layer blood vessels can be observed clearly.

In the blood vessel index value acquisition unit 76, the blood vessel index values of the surface layer blood vessels are acquired in the case of the surface layer blood vessel observation mode, and the blood vessel index values of the middle-deep layer blood vessels are acquired in the case of the middle-deep layer blood vessel observation mode. In the blood vessel parameter calculation unit 78, a the blood vessel parameter can be accurately calculated by using the blood vessel index values of the surface layer blood vessels obtained in the surface layer blood vessel observation mode and the blood vessel index values of the middle-deep layer blood vessels obtained in the middle-deep layer blood vessel observation mode.

### [Fifth Embodiment]

In the fifth embodiment, the observation target is illuminated using a broadband light source, such as a xenon lamp, and a rotation filter instead of the four-color LEDs 20a to 20d illustrated in the above respective embodiments. Additionally, the observation target is imaged by a monochrome imaging sensor instead of the color imaging sensor 38. The others are the same as those of the first embodiment.

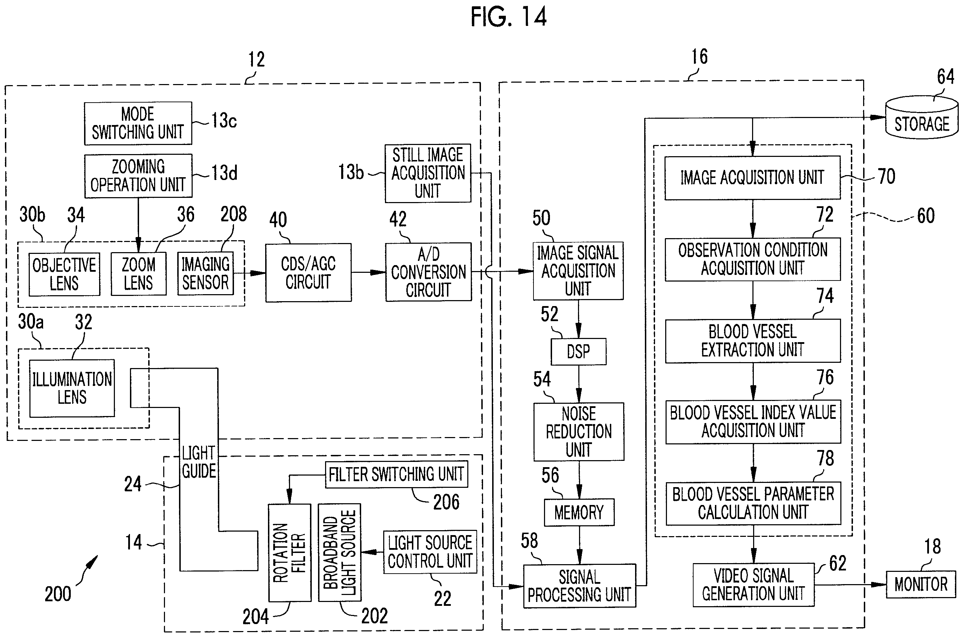

As illustrated in Fig. 14, in an endoscope system 200 of the fifth embodiment, in the light source device 14, a broadband light source 202, a rotation filter 204, and a filter switching unit 206 are provided instead of the four-color LEDs 20a to 20d. Additionally, the imaging optical system 30b is provided with a monochrome imaging sensor 208, which is not provided with a color filter, instead of the color imaging sensor 38.

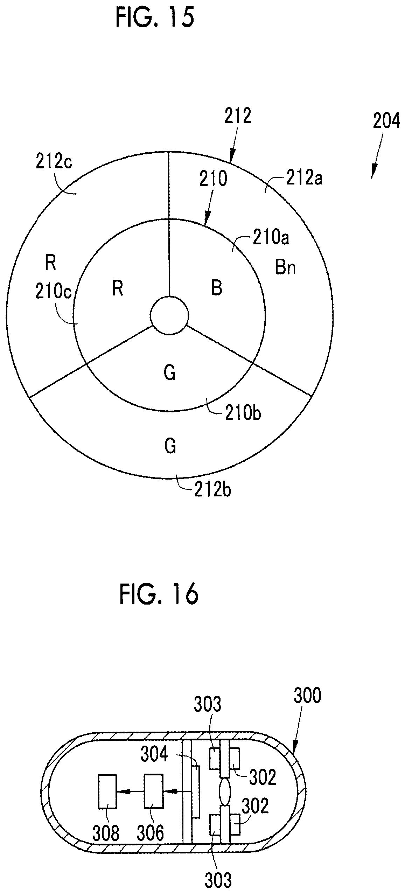

The broadband light source 202 is a xenon lamp, a white LED, or the like, and emits white light of which the wavelength range ranges from blue to red. The rotation filter 204 includes a normal mode filter 210 provided inside, and a special mode filter 212 provided outside (refer to Fig. 15). The filter switching unit 206 moves the rotation filter 204 in a radial direction, inserts the normal mode filter 210 of the rotation filter 204 into an optical path for white light in a case where the normal mode is set by a mode switchover switch (not illustrated), and inserts the special mode filter 212 of the rotation filter 204 into the optical path for white light in a case where the special mode is set.

As illustrated in Fig. 15, a B filter 210a, a G filter 210b, and an R filter 210c is provided in a circumferential direction in the normal mode filter 210. The B filter 210a allows blue light in white light be transmitted therethrough. The G filter 210b allows green light in white light to be transmitted therethrough. The R filter 210c allows red light in white light to be transmitted therethrough. Hence, in the normal mode, the observation target is alternately illuminated with blue light, green light, and red light as the rotation filter 204 rotates.

A Bn filter 212a, a G filter 212b, and an R filter 212c are provided in the circumferential direction in the special mode filter 212. The Bn filter 212a allows blue narrowband light of a specific wavelength range in white light to be transmitted therethrough. The G filter 212b allows green light in white light to be transmitted therethrough. The R filter 212c allows red light in white light to be transmitted therethrough. Hence, in the special mode, the observation target are alternately illuminated with narrowband light, green light, and red light as the rotation filter 204 rotates.

In addition, in addition to the above respective filters 212a to 212c, a V filter that allows purple light in white light to be transmitted therethrough and a B filter that allows blue light in white light to be transmitted therethrough may be provided in the special mode filter 212. As in the above first embodiment, in a case where the purple light V and the blue light B are emitted sequentially in the special mode, the special mode filter 212 may be provided with the V filter and the B filter.

In the endoscope system 200, in the normal mode, the observation target is imaged by the monochrome imaging sensor 208 whenever the observation target is illuminated with blue light, green light, and red light. Accordingly, image signals of three colors of RGB can be obtained. Then, a normal observation image is created by the same method as the above first embodiment on the basis of the RGB-color image signals.

Meanwhile, in the special mode, the observation target is imaged by the monochrome imaging sensor 208 whenever the observation target is illuminated with blue narrowband light, green light, and red light. Accordingly, a Bn image signal, a G image signal, and a R image signal can be obtained. A special observation image is generated by the same method as the first embodiment, on the basis of the Bn image signal, the G image signal, and the R image signal.

### [Sixth Embodiment]

In the above respective embodiments, the invention is implemented by the endoscope system 10 or the endoscope system 200 that inserts the endoscope 12 provided with the imaging sensor 38 into a subject, thereby performing observation. However, in the sixth embodiment, the invention is implemented in a capsule endoscope system. The capsule endoscope system has, for example, at least a capsule endoscope 300 illustrated in Fig. 16, and a processor device (not illustrated). The capsule endoscope 300 includes a light source 302, a light source control unit 303, an imaging sensor 304, an image signal acquisition processing unit 306, and a transmitting and receiving antenna 308. The light source 302 is configured similar to the light source 20 of the endoscope system 10, and emits illumination light through the control of the light source control unit 303. The image signal acquisition processing unit 306 functions as the image signal acquisition unit 50, the DSP 52, the noise reduction unit 54, and the signal processing unit 58. The processor device of the capsule endoscope system is configured similar to the processor device 16 of the endoscope system 10, and also functions as the image processing unit 60. The endoscopic image generated by the image signal acquisition processing unit 306 is transmitted to the processor device via the transmitting and receiving antenna 308. In the processor device, the first blood vessel index value in the first observation condition is acquired from the received endoscopic image, the second blood vessel index value in the second observation condition is acquired from the received endoscopic image, and the blood vessel parameter is calculated using the first blood vessel index value and the second blood vessel index value.

### Explanation of References

- 10, 200:: endoscope system
- 12:: endoscope
- 14:: light source device
- 16:: processor device
- 70:: image acquisition unit
- 74:: blood vessel extraction unit
- 76:: blood vessel index value acquisition unit
- 78:: blood vessel parameter calculation unit
- 102:: blood vessel index value correction unit
- 112:: distance acquisition unit
- 122:: determination unit

## Claims

1. An endoscope system comprising:
an image acquisition unit that acquires an image obtained by imaging an observation target by an endoscope;
a blood vessel index value acquisition unit that acquires a first blood vessel index value in a first observation condition and a second blood vessel index value in a second observation condition different from the first observation condition, as blood vessel index values of blood vessels of an observation target from the image, thereby acquiring at least two or more blood vessel index values including the first blood vessel index value and the second blood vessel index value; and
a blood vessel parameter calculation unit that calculates a blood vessel parameter, using the first blood vessel index value and the second blood vessel index value.

2. The endoscope system according to claim 1,
wherein the blood vessel parameter calculation unit performs calculation while performing weighting based on the first blood vessel index value and the second blood vessel index value on the first blood vessel index value and the second blood vessel index value.

3. The endoscope system according to claim 2,
wherein the blood vessel index value acquisition unit acquires at least three or more blood vessel index values including the first blood vessel index value, the second blood vessel index value, and a specific blood vessel index value, and
wherein the blood vessel parameter calculation unit performs calculation while performing weighting on only blood vessel index values other than the specific blood vessel index value among the three or more blood vessel index values.

4. The endoscope system according to claim 2 or 3, further comprising:
a blood vessel index value correction unit that corrects the first blood vessel index value with a first correction value based on the first observation condition and corrects the second blood vessel index value with a second correction value based on the second observation condition,
wherein the blood vessel parameter calculation unit performs calculation while performing weighting on the first blood vessel index value corrected with the first correction value and the second blood vessel index value corrected with the second correction value.

5. The endoscope system according to claim 4, further comprising:
a distance acquisition unit that acquires a first observation distance, which is an observation distance from the observation target, and a second observation distance different from the first observation distance, from the image,
wherein the first correction value is a correction value in a case where the first observation condition is the first observation distance, and
wherein the second correction value is a correction value in a case where the second observation condition is the second observation distance.

6. The endoscope system according to claim 1,
wherein the blood vessel parameter calculation unit calculates the blood vessel parameter by four arithmetic operations.

7. The endoscope system according to any one of claims 1 to 6, further comprising:
a determination unit that determines a state of a mucous membrane of the observation target, using the blood vessel parameter.

8. The endoscope system according to claim 7,
wherein the determination unit determines the state of the mucous membrane of the observation target as any of three or more types of states including normality, adenoma, and cancer, using the blood vessel parameter.

9. The endoscope system according to claim 8,
wherein the determination unit determines the state of the mucous membrane of the observation target as any one of states including normality, hyperplastic polyp, SSA/P, adenoma, laterally spreading tumor, and cancer, using the blood vessel parameter.

10. The endoscope system according to claim 8 or 9,
wherein the determination unit further determines a stage of cancer in a case where the state of the mucous membrane of the observation target is determined as the state of cancer.

11. The endoscope system according to any one of claims 1 to 10,
wherein the blood vessel index value acquisition unit acquires at least two or more blood vessel index values from a number of blood vessels, a number of branches of blood vessels, a branch angle of blood vessels, a distance between branch points, a number of intersections of blood vessels, a thickness of blood vessels, a change in thickness of blood vessels, a degree of complexity of a change in thickness of blood vessels, a length of blood vessels, an interval of blood vessels, a depth of blood vessels, a height difference of blood vessels, an inclination of blood vessels, an area of blood vessels, a density of blood vessels, a contrast of blood vessels, a color of blood vessels, a change in color of blood vessels, a degree of meandering of blood vessels, a blood concentration of blood vessels, a degree of oxygen saturation of blood vessels, a proportion of arteries, a proportion of veins, a concentration of am input coloring agent, a traveling pattern of blood vessels, or a blood flow rate of blood vessels.

12. The endoscope system according to claim 11,
wherein the first blood vessel index value is the same type as the second blood vessel index value, and the values are different from each other.

13. The endoscope system according to claim 11,
wherein the first blood vessel index value is of a type different from the second blood vessel index value.

14. The endoscope system according to any one of claims 1 to 13,
wherein the first observation condition and the second observation condition are at least any one of an observation distance from the observation target, a zoom magnification factor of the endoscope, an observation mode in which special light is used, or a coloring agent dispersed in the observation target.

15. A method of operating an endoscope system comprising:
acquiring an image obtained by imaging an observation target by an endoscope by an image acquisition unit;
acquiring a first blood vessel index value in a first observation condition and a second blood vessel index value in a second observation condition different from the first observation condition, as blood vessel index values of blood vessels of an observation target from the image, thereby acquiring at least two or more blood vessel index values including the first blood vessel index value and the second blood vessel index value by a blood vessel index value acquisition unit; and
calculating a blood vessel parameter, using the first blood vessel index value and the second blood vessel index value, by a blood vessel parameter calculation unit.
